# EUROPÄISCHE PATENTSCHRIFT

(11) **EP 0 371 408 B1**
(45) Veröffentlichungstag und Bekanntmachung des Hinweises auf die Patenterteilung: **22.06.1994**
(21) Anmeldenummer: 89121754.9
(22) Anmeldetag: 24.11.1989
(51) Int. Cl.: C12N 11/14

(54) **Biokatalysatoren und Verfahren zu ihrer Herstellung**
Biocatalysts and process for their preparation
Biocatalyseurs et leur procédé de préparation

(30) Priorität: 28.11.1988 CH 4403/88
(43) Veröffentlichungstag der Anmeldung: 06.06.1990
(73) Patentinhaber: CIBA-GEIGY AG, 4002 Basel (CH)
(72) Erfinder: Fauquex, Pierre François, Dr., CH-4127 Birsfelden (CH); Sedelmeier, Gottfried, Dr., D-7801 Schallstadt (CH)
(74) Vertreter: Zumstein, Fritz, Dr.

(56) Entgegenhaltungen:
- EP-A- 0 034 293
- EP-A- 0 035 121
- EP-A- 0 079 595
- EP-A- 0 278 556
- DE-A- 2 727 143
- DE-A- 2 835 875
- DE-A- 3 417 899
- GB-A- 2 128 620
- GB-A- 2 175 917

## Beschreibung

Die vorliegende Erfindung betrifft perlförmige Biokatalysatoren mit hoher mechanischer Festigkeit, Verfahren zu ihrer Herstellung und Verfahren zur Umsetzung von organischen Substanzen mit Hilfe der erfindungsgemässen Biokatalysatoren.

### Hintergrund der Erfindung

Der Begriff Biokatalyse umfasst im weitesten Sinne alle Formen der Katalyse, bei denen die aktivierende Substanz ein biologisches System ist, z.B. ganze Zellen, Zellfragmente oder Zellorganellen, oder aus einem biologischen System stammt, z.B. Enzyme. Enzyme sind Verbindungen, im allgemeinen Proteine oder Glykoproteine, die spezifisch biochemische Reaktionen beschleunigen, indem sie die Aktivierungsenergie herabsetzen. Im folgenden bezeichnet der Ausdruck Biokatalysator nicht nur die aktivierende Substanz selbst, sondern kann gegebenenfalls auch Komponenten umfassen, die zur Immobilisierung, Haltbarkeit, Regeneration etc. der aktivierenden Substanz verwendet wurden.

Immobilisierte, d.h. in ihrer Beweglichkeit eingeschränkte Biokatalysatoren eignen sich vor allem für industrielle Zwecke. Durch die Immobilisierung wird es möglich, die katalysierten Prozesse kontinuierlich und wiederholt durchzuführen, hohe Biokatalysatordichten zu schaffen und aufrechtzuerhalten, hohe Zeit-Raum-Ausbeuten zu erzielen und die Kosten für die Gewinnung des Produktes aus der Reaktionslösung zu senken.

Das biokatalytische Material kann zur Immobilisierung beispielsweise in polymere Hüllsubstanzen (Matrix), in Kapseln bzw. Fasern aus semipermeablen Membranen oder hinter Ultrafiltrationsmembranen eingeschlossen, mit bi- bzw. multifunktionellen Reagenzien quervernetzt oder durch Adsorption, durch ionische oder kovalente Bindung an Träger aus anorganischem Material oder natürlichen bzw. synthetischen Polymeren fixiert sein. Auch eine Kombination dieser Methoden ist möglich.

Bei der Immobilisierung muss einerseits der grösstmögliche Erhalt der biokatalytischen Aktivität und andererseits hohe mechanische Festigkeit und chemische Stabilität, aber gleichzeitig auch gute Permeabilität des Biokatalysators gewährleistet sein. Eine gebräuchliche Immobilisierungsmethode ist beispielsweise der Einschluss ("entrapment") des biologisch aktiven Materials in eine Matrix aus natürlichen Polymeren wie Cellulose, Agar, Gelatine u.a. oder aus synthetischen Polymeren wie Polyacrylamid, Polyurethanen, Epoxidharzen u.a.. Besonders schonend ist die Immobilisierung durch ionotrope Gelbildung (ionische Quervernetzung der Matrixsubstanzen), für die sich als Hüllmaterial beispielsweise natürliche Polyanionen wie Alginat, Pektin, Carrageenan u.a. eignen. Durch Eintropfen oder Einsprühen einer Suspension aus enzymatisch aktivem Material und einer wässrigen Lösung der Matrixsubstanz in eine wässrige Vernetzerlösung, die multivalente Gegenionen enthält, also beispielsweise Ca²⁺, Co²⁺, Zn²⁺, Fe²⁺, Fe³⁺, Al³⁺ u.a., kommt es zur Chelatisierung und Bildung von perlförmigen Biokatalysatoren.

Bei der ionotropen Gelbildung durch organische Polymere ergeben sich in der Regel nur relativ weiche, mechanisch instabile Immobilisate, die zur Quellung und Deformation tendieren, so dass sich beispielsweise bei ihrer Verwendung in Füllkörperreaktoren, wie sie für enzymatische Reaktionen häufig industriell eingesetzt werden, Schwierigkeiten ergeben. Härtungsmethoden durch Nachbehandlung der Immobilisate oder durch Vorbehandlung des biologisch aktiven Materials vor der Zugabe zum Gelierungsmittel jeweils mit multifunktionellen Vernetzungsagentien wie Polyaldehyden, z.B. Glutaraldehyd, oder Isocyanaten, z.B. Hexamethylendiisocyanat, können wegen ihrer zell- und/oder enzymschädigenden Wirkung nur sehr begrenzt eingesetzt werden. Eine Alternative ist die Verwendung von anorganischen Substanzen, z.B. wie in der deutschen Offenlegungsschrift DE 35 20 001 beschriebenen Verfahren, das die Immobilisierung enzymatischen Materials mittels Silikasol und Alginat offenbart. Auch Trocknung und Schrumpfung der Biokatalysatorperlen wird zur Härtung eingesetzt, da die Perlen nach Wiederbefeuchtung ihr ursprüngliches Feuchtvolumen nicht wieder annehmen und härter bleiben. Die deutsche Offenlegungsschrift DE 28 35 875 beschreibt ein Verfahren der ionotropen Gelbildung unter Verwendung von Polyanionen, z.B. Natriumalginat, und beispielsweise Calciumionen als Vernetzer, bei dem die mit enzymatischer Substanz beladenen Biokatalysatorperlen bei einer Temperatur bis zu 80°C getrocknet werden. Solche Trocknungsschritte können jedoch immer dann nicht durchgeführt werden, wenn hochempfindliche Enzyme oder Enzymsysteme, insbesondere lebende Zellen, immobilisiert werden sollen, da die hohen Temperaturen bei der Trocknung bzw. der Trockenzustand die Zellen oder Enzyme inaktivieren.

Ein weiterer Nachteil der durch ionotrope Gelbildung hergestellten Calcium-Alginat-Immobilisate ist neben den schlechten mechanischen Eigenschaften auch die Beschränkung der möglichen Reaktionsmedien bei der Weiterverarbeitung. Solche Immobilisate sind beispielsweise chemisch instabil bei Anwesenheit von in den üblichen Pufferlösungen enthaltenen Ionen, wie Natrium oder Kalium. In solchen Elektrolytlösungen, besonders wenn sie hochkonzentriert sind, werden zum Beispiel die gelfestigenden Calciumionen in Calcium-Alginat-Gelen verdrängt, weil Natrium- bzw. Kaliumionen um das Alginatanion konkurrieren. Auch Phosphationen führen zur allmählichen Auflösung von Calcium-Alginat-Biokatalysatorperlen, da Phosphate mit Calcium reagieren und dem Alginat das strukturfestigende Agens entziehen. Die Auflösung kann verhindert werden, wenn als Matrixsubstanzen Polykationen, z.B. Chitosan, verwendet werden, die mit mehrwertigen Anionen wie [Fe(CN)₆]³⁻, [Fe(CN)₆]⁴⁻, Polyphosphaten u.a. vernetzt werden. Die deutschen Patentschriften DE 30 05 632 und DE 30 05 633 beschreiben die Herstellung von Biokatalysatorperlen unter Verwendung von Chitosan und K₃(Fe(CN)₆). Das Verfahren nach DE 30 05 632 enthält zur Härtung der Perlen einen Trocknungsschritt bei einer Temperatur bis zu 80°C. Um die Immobilisierung hochempfindlicher enzymatischer Substanz zu ermöglichen, ist dieser Trocknungsschritt im Verfahren nach DE 30 05 633 nicht enthalten, was jedoch zu geringerer mechanischer Festigkeit der Biokatalysatorperlen führt.

### Gegenstand der Erfindung

Es ist Aufgabe der vorliegenden Erfindung, perlförmige Biokatalysatoren zur Verfügung zu stellen,
· die hohe mechanische Festigkeit aufweisen,
· deren Stabilität auch bei Verwendung der üblichen biologischen Lösungen und Puffersystemen gewährleistet ist, und
· die die enzymatische Aktivität hochempfindlichen enzymatisch aktiven Materials weiterhin gewährleisten.

Weiterhin ist es Aufgabe der Erfindung, geeignete Verfahren zur Herstellung solcher Biokatalysatoren aufzuzeigen.

Als besonders geeignet für die Lösung dieser Aufgaben erweisen sich perlförmige Biokatalysatoren, die enzymatisch aktives Material, einen kationischen Polyelektrolyten, vorzugsweise Chitosan, mehrwertige Anionen und Kieselsäure enthalten. Derartige Biokatalysatorperlen werden in einem schonenden Immobilisierungsverfahren durch ionotrope Gelbildung und mittels Verwendung von Kieselsäure in fester Form hergestellt.

### Beschreibung der Erfindung

Die vorliegende Erfindung betrifft perlförmige Biokatalysatoren mit hoher mechanischer Festigkeit umfassend enzymatisch aktives Material, einen kationischen Polyelektrolyten und mehrwertige Anionen, dadurch gekennzeichnet, dass die erfindungsgemässen Biokatalysatoren Kieselsäure enthalten.

Die erfindungsgemässen perlförmigen Biokatalysatoren sind durch hohe mechanische Festigkeit gekennzeichnet, was auf die Ausbildung einer Dual-Gelmatrix-Struktur zurückzuführen ist. Sie weisen daher beispielsweise beim Füllen eines Reaktionsgefässes gute Packungseigenschaften auf und können als Füllmaterial etwa für Festbettreaktoren verwendet werden. Aufgrund der milden Immobilisierungsbedingungen, die keinen Trocknungsschritt bzw. eine Behandlung mit schädigenden Substanzen einschliessen, eignen sich die erfindungsgemässen Biokatalysatoren vor allem für hochempfindliches enzymatisches Material, insbesondere für lebende Zellen. Die Herstellung der perlförmigen Biokatalysatoren gemäss der Erfindung mittels Verwendung von Kieselsäure in fester Form hat gegenüber den aus dem Stand der Technik bekannten Verfahren die Vorteile, dass zeit- und kostenaufwendige Verfahren zur Vorbereitung der beim Härtungsschritt eingesetzten Vernetzungsagentien vermieden werden. Weiterhin können die erfindungsgemässen Biokatalysatoren in Puffersystemen eingesetzt werden, in denen Ionen enthalten sind, die zur Auflösung von Alginat-Gelen führen. So können die erfindungsgemässen Biokatalysatoren beispielsweise in Lösungen eingesetzt werden, die Phosphationen enthalten, z.B. in dem sehr preiswerten und daher für industrielle Zwecke vorteilhaften Tripolyphosphatpuffer. Die erfindungsgemässen perlförmigen Biokatalysatoren eignen sich für die Umsetzung organischer Substanzen. Da sie für Reaktionen verwendet werden können, die hohe Elektrolytkonzentrationen erfordern, eignen sich beispielsweise erfindungsgemässe Biokatalysatorperlen, die ein Bakterium des Genus Proteus bzw. eine Oxidoreduktase als enzymatisch aktives Material enthalten, für die Herstellung von α-Hydroxycarbonsäuren aus α-Ketocarbonsauren, z.B. von 2-(R)-Hydroxy-4-phenylbuttersäure aus 2-Oxo-4-phenylbuttersäure.

Die erfindungsgemässen perlförmigen Biokatalysatoren weisen hohe mechanische Festigkeit auf. Unter hoher mechanischer Festigkeit wird beispielsweise hohe Druckfestigkeit verstanden, d.h. dass erfindungsgemässe Biokatalysatorperlen mit einer durchschnittlichen Grösse von ungefähr 3 mm bei einer Belastung von 0,01 bis 0,03 N, insbesondere von 0,02 N, nach ein bis fünf Minuten eine maximale Deformation von 30 bis 50 »m, insbesondere von 40 »m, aufweisen und nach 10 bis 20 Minuten, insbesondere 15 Minuten, keine weitere Deformationszunahme mehr zeigen.

Die Art des enzymatisch aktiven Materials, das die erfindungsgemässen Biokatalysatoren umfassen, ist bestimmt durch die Reaktion, für die die Biokatalysatoren verwendet werden sollen. Es besteht vorzugsweise aus ganzen Zellen, Zellfragmenten oder Zellorganellen eines Mikroorganismus, aus Enzymen oder aus einer beliebigen Kombination dieser Arten des enzymatisch aktiven Materials.

Der Mikroorganismus kann prokaryontischer oder eukaryontischer Natur sein. Geeignete Mikroorganismen sind beispielsweise
- ein Bakterium, z.B. ein Milchsäurebakterium (Leuconostoc, Streptococcus, Lactobacillus), Corynebacterium, Streptomyces, Pseudomonas, Xanthomonas, Bacillus, Clostridium, Escherichia, Proteus u.a.,
- ein Pilz, z.B. Mucor, Aspergillus, Penicillium, insbesondere Hefen wie etwa Saccharomyces, Candida u.a.,
- eine Alge, z.B. Chlorella, Curvularia u.a., oder
- eine pflanzliche Zelle, z.B. Catharanthus roseus, Daucus carota, Digitalis lanata, Morinda citrifolia u.a..

Der Einsatz von ganzen Zellen, Zellfragmenten oder Zellorganellen, d.h. von Membranen umhüllten bzw. aus Membransystemen bestehenden Zelluntereinheiten (Chloroplasten, Thylakoide, Mitochondrien u.a.), als enzymatisch aktives Material ist vorteilhaft, wenn die zu katalysierende Reaktion ein Multienzymsystem verlangt, z.B. wenn Cofaktoren (Pyridin- oder Flavinnucleotide usw.) benötigt werden, oder wenn anstelle eines katabolischen Produktes ein biosynthetisches, z.B. ein sekundäres, Produkt hergestellt werden soll, wozu komplizierte Stoffwechselketten erforderlich sind.

Als enzymatisch aktives Material eignen sich auch isolierte Enzyme. Typische Beispiele solcher Enzyme sind Oxidoreduktasen (Lactatdehydrogenase, Alkoholdehydrogenase, Glucoseoxidase u.a.), Transferasen (Hexokinase, Glutamintransaminase u.a.), Lyasen (Fumarase, Aspartase u.a.), Isomerasen (Glucoseisomerase, Mannoseisomerase u.a.), Ligasen (Glutathionsynthetase, NAD-Synthetase u.a.) usw..

Als enzymatisch aktives Material können die erfindungsgemässen Biokatalysatoren auch eine beliebige Kombination der beschriebenen Arten des enzymatischen Materials, beispielsweise eine Kombination von ganzen Zellen zweier oder mehrerer Spezies oder Stämme von Mikroorganismen oder eine Kombination von ganzen Zellen und Enzymen, umfassen. Grundgedanke ist die Komplettierung der biokatalytischen Eigenschaften einer Zelle mit zusätzlichen Enzymen, die in der Zelle nicht oder in zu geringer Menge verfügbar sind.

Als enzymatisches Material besonders bevorzugt sind ganze Zellen eines Bakteriums oder einer Hefe, vorzugsweise eines Bakteriums des Genus Proteus, insbesondere eines Bakteriums der Spezies Proteus vulgaris und/oder der Spezies Proteus mirabilis. Besonders bevorzugt ist die Spezies Proteus vulgaris, z.B. Proteus vulgaris ATCC 9484. Ebenfalls besonders bevorzugt ist enzymatisch aktives Material, das eine Oxidoreduktase enthält oder eine Oxidoreduktase ist.

Der kationische Polyelektrolyt ist ein natürliches oder synthetisches Polymer oder ein natürliches Polymer nach chemischer Modifikation, beispielsweise ein Polymer mit protonisierten Aminogruppen, bevorzugt Chitosan ([2-Amino-2-deoxy-(1→4)-β-D-glucopyranan; Poly-(1,4-β-D-glucopyranosamin]). Chitosan ist ein lineares Polymer mit hohem Molekulargewicht aus Glucosamin-Untereinheiten, das durch teilweise Deacetylierung mit Hilfe von konzentrierter Lauge und Hitze aus Chitin gewonnen wird. Chitin ist in der Natur zum Beispiel in marinen Invertebraten, Insekten und Pilzen weit verbreitet. Zur Produktion von Chitosan wird im allgemeinen Chitin aus Krabben, Garnelen, Hummern usw. eingesetzt. Die erfindungsgemässen perlförmigen Biokatalysatoren umfassen vorzugsweise Chitosan mit einem Molekulargewicht von etwa 50'000 bis etwa 3'000'000.

Als mehrwertige Anionen eignen sich mehrwertige anorganische oder mehrwertige organische Anionen. Die anorganischen Anionen sind vorzugsweise Orthophosphat, Metaphosphate, z.B. Hexametaphosphat, Pyrophosphate, Polyphosphate, z.B. Tri-, Tetra- oder Octapolyphosphat, oder Cyanoferrate, z.B. [Fe(CN)₆]⁴⁻ oder [Fe(CN)₆]³^{⁻}. Besonders bevorzugt ist Tripolyphosphat. Die organischen Anionen sind vorzugsweise polymere organische Carboxylate, Sulfonate oder Hydroxyverbindungen.

Bei der Kieselsäure eignet sich vorzugsweise eine gefällte Kieselsäure, besonders bevorzugt eine gefällte Kieselsäure mit einer durchschnittlichen Korngrösse im Bereich von 5 bis 50 »m und einem durchschnittlichen Schüttgewicht im Bereich von 5 bis 100 g/100 ml, insbesondere von etwa 20 bis 50 g/100 ml.

Weiterhin betrifft die vorliegende Erfindung ein Verfahren zur Herstellung von erfindungsgemässen perlförmigen Biokatalysatoren mit hoher mechanischer Festigkeit, dadurch gekennzeichnet, dass enzymatisch aktives Material mit gefällter Kieselsäure in fester Form, einer wässrigen Pufferlösung und der wässrigen Lösung eines kationischen Polyelektrolyten unter Vermeidung von Schaumbildung zu einer Suspension vermischt wird, die Suspension in ein wässriges Vernetzerbad enthaltend mehrwertige Anionen tropfenweise eingebracht wird und die entstandenen Biokatalysatorperlen im Vernetzerbad geschrumpft und verfestigt und gegebenenfalls vom Vernetzerbad getrennt werden.

Die Vermeidung von Schaumbildung bei der Herstellung der Suspension aus enzymatisch aktivem Material, gefällter Kieselsäure in fester Form, wässriger Pufferlösung und der wässrigen Lösung eines kationischen Polyelektrolyten ist von entscheidender Bedeutung, da sonst als Folge Katalysatorperlen mit schlechteren mechanischen Eigenschaften entstehen. Bevorzugt wird Schaumbildung vermieden, indem die Vermischung der verschiedenen Komponenten in Gegenwart der Kieselsäure erfolgt. Ansonsten ist die Reihenfolge, in der die Substanzen zusammengebracht werden, beliebig. Bevorzugt wird das enzymatische Material zunächst mit der Kieselsäure in fester Form gemischt, erst dann in wässriger Pufferlösung aufgenommen und anschliessend mit der Lösung des kationischen Polyelektrolyten gemischt.

Bei der erfindungsgemässen Herstellung der Biokatalysatorperlen wird enzymatisches Material wie oben beschrieben verwendet, d.h. ganze Zellen, Zellfragmente oder Zellorganellen eines Mikroorganismus oder Enzyme oder eine Kombination davon. Ganze Zellen können dabei lebend, tot oder beispielsweise lyophilisiert oder dehydriert vorliegen. Die Anzucht der Zellen in einem Nährmedium, das alle für die Aufrechterhaltung des Zellstoffwechsels und für die Vermehrung notwendigen organischen und anorganischen Bestandteile (C-Quelle, N-Quelle, Spurenelemente, Wuchsstoffe etc.) enthält, und die Zellfragmentierung zur Herstellung von Zellfragmenten bzw. die Isolierung der Zellorganellen erfolgt nach an sich bekannten Methoden. Zu immobilisierende Enzyme liegen beispielsweise gelöst, dispergiert, suspendiert, emulgiert oder getrocknet vor. Das genannte und im vorausgegangenen näher definierte enzymatisch aktive Material wird für das erfindungsgemässe Verfahren beispielsweise als Zell-, Zellfragment- oder Zellorganellsediment, -filtrat oder -suspension oder als Enzym in wässriger Lösung eingesetzt. Im Zusammenhang mit der vorliegenden Beschreibung bedeutet der Ausdruck "in wässriger Lösung", dass die Lösung zusätzlich noch anorganische oder organische Salze und dergleichen enthalten kann. Es kann sich beispielsweise um einen gebräuchlichen biologischen Puffer wie Acetat-, Phosphat-, Trispuffer und dergleichen handeln.

Als wässrige Pufferlösung eignen sich beispielsweise biologische Puffer in einem pH-Bereich von pH 3 bis 7, bevorzugt von etwa pH 5, z.B. Phosphat-, Citrat- oder Acetatpuffer.

Die bei dem erfindungsgemässen Verfahren verwendbaren kationischen Polyelektrolyten sind im vorangegangenen beschrieben. Bevorzugt ist Chitosan. Chitosan muss für die Verwendung zur Herstellung der Biokatalysatorperlen zunächst in wässrige Lösung gebracht werden, um integraler Bestandteil des Immobilisats zu werden. Dazu wird Chitosan, das in nicht-protonisierter Form wasserunlöslich ist, in verdünnten wassrigen, insbesondere organischen, Säuren, beispielsweise in Ameisensäure, Essigsäure, Brenztraubensäure, Aepfelsäure, Zitronensäure und dergleichen, bevorzugt in Essigsäure, bei einem pH Wert ≦ 7, bevorzugt pH 4 bis 5,5, aufgenommen und gegebenenfalls auf etwa 60°C erhitzt, um den Lösungsprozess zu unterstützen. Die Chitosanlösung wird anschliessend zur Entfernung unlöslicher Bestandteile filtriert. Für die Herstellung der erfindungsgemässen Biokatalysatorperlen wird vorzugsweise eine wassrige Lösung von Chitosan mit einer Viskosität im Bereich von 1'000 bis 20'000 cP in einer Konzentration im Bereich von 0,5 bis 5 % (w/w) eingesetzt. Besonders bevorzugt ist die Verwendung einer wässrigen Lösung von hochviskosem Chitosan mit einer Viskosität von ca. 10'000 cP in einer Konzentration von ca. 1,4 % (w/w).

Die verwendeten Substanzen werden vorzugsweise so eingesetzt, dass das Verhältnis der Gewichtsteile von Kieselsäure zu kationischem Polyelektrolyt in einem Bereich von 1,5 bis 50 zu 1, besonders bevorzugt bei ungefähr 15 zu 1, liegt. Bevorzugte Endkonzentrationen von Kieselsäure in der oben beschriebenen Suspension liegen in einem Bereich von 2 bis 15 % (w/w), z.B. bei 10 %. Bevorzugte Endkonzentrationen von kationischem Polyelektrolyt in der oben beschriebenen Suspension sind 0,3 bis 1,5 % (w/w), insbesondere um 0,7 %.

Die Kieselsäure, die zur Herstellung der erfindungsgemässen Biokatalysatorperlen verwendet wird, ist oben beschrieben. Sie wird in fester Form eingesetzt.

Beim Vernetzerbad handelt es sich um die wässrige Lösung eines mehrwertigen Anions. Die möglichen Anionen sind im vorangegangenen beschrieben. Sie werden in Form der Säuren oder als Salze für das Vernetzerbad eingesetzt, z.B. als Alkalisalze, beispielsweise Natrium- oder Kaliumsalze, ferner auch als Ammoniumsalze. Bevorzugt ist eine wässrige Lösung, für die Tripolyphosphorsäure oder ein Salz davon, z.B. Natrium- oder Kalium-tripolyphosphat, eingesetzt wird. Im Vernetzerbad liegen die genannten Verbindungen beispielsweise in einer Konzentration im Bereich von 0,5 bis 10 % (w/v), bevorzugt von 1,5 bis 3 % (w/v), vor. Der pH-Wert der Vernetzerlösung liegt in einem Bereich von pH 5 bis 10, bevorzugt von etwa pH 8.

Die Suspension aus enzymatisch aktivem Material, Kieselsäure, Pufferlösung und Polykation wird unter Rühren tropfenweise in die Vernetzerlösung eingebracht, zweckmässig so, dass das Volumen des Vernetzerbades mindestens etwa das Dreifache des Volumens der Suspension beträgt. Die tropfenweise Zugabe erfolgt unter Verwendung einer Düse mit einer kleinen Oeffnung, beispielsweise einer Injektionsspritze, einer spritzenähnlichen Injektionseinrichtung, einer porösen Platte oder eines ähnlichen geeigneten Mittels. Die Tropfen werden gegebenenfalls von der Spitze der Düse unter Verwendung von komprimierter Luft, komprimiertem Stickstoff oder dergleichen abgeblasen. Die Suspension kann auch beispielsweise unter Verwendung einer Atomisiervorrichtung, vorzugsweise einer Druckatomisiervorrichtung, in die Vernetzerlösung eingesprüht werden.

Die nach dem oben beschriebenen Verfahren erhaltenen Biokatalysatorperlen verbleiben zur Schrumpfung und Härtung für mindestens etwa eine Stunde, z.B. über Nacht, im Vernetzerbad. Gegebenenfalls werden sie durch die üblichen, zur Trennung von festen und flüssigen Phasen geeigneten Methoden wie Dekantieren, Filtration usw. vom Vernetzerbad getrennt und vor der weiteren Verwendung einem physiologischen Waschprozess, beispielsweise mit physiologischer Kochsalzlösung, unterworfen.

Perlförmige Biokatalysatoren mit hoher mechanischer Festigkeit, die nach den erfindungsgemässen Verfahren hergestellt werden, sind ebenfalls Gegenstand der vorliegenden Erfindung.

Die Erfindung betrifft ausserdem Verfahren zur Umsetzung organischer Substanzen, dadurch gekennzeichnet, dass für die Umsetzung perlförmige Biokatalysatoren gemäss der vorliegenden Erfindung eingesetzt werden.

Je nach Art des immobilisierten enzymatisch aktiven Materials ist die Produktion einer breiten Palette von Produkten möglich, die durch Fermentationsprozesse, im Primärstoffwechsel, durch mikrobielle Transformation usw. entstehen, beispielsweise von
- Chemikalien, z.B. Zitronensäure durch Aspergillus niger, Ethanol durch Zymomonas mobilis, L-Aepfelsäure durch Fumarase, H₂ durch Chlorella-Clostridien-Coimmobilisate,
- Nahrungsmitteln, z.B. Sojasauce durch Pediococcus halophilus,
- Enzymen, z.B. Amylasen durch Aspergillus niger,
- Medikamente, z.B. Penicilline durch Penicillium chrysogenum, oder
- Aminosäuren, z.B. L-Asparaginsäure durch L-Aspartase.

Bevorzugt sind Verfahren zur Herstellung von α-Hydroxycarbonsäuren aus α-Ketocarbonsäuren mit Hilfe von erfindungsgemässen Biokatalysatoren, deren enzymatisch aktives Material eine Oxidoreduktase enthält, eine Oxidoreduktase ist oder aus ganzen Zellen eines Bakteriums des Genus Proteus besteht, insbesondere der Spezies Proteus vulgaris und/oder Proteus mirabilis, bevorzugt Proteus vulgaris. Die Reduktion des Substrats erfolgt dabei durch die sogenannte finale Reduktase, z.B. eine substratspezifische Dehydrogenase. Im allgemeinen werden die Reduktionsäquivalente, die von der finalen Reduktase benötigt werden, von einem Coenzym geliefert, z.B. von Pyridinnucleotiden wie Nicotinamidadenindinucleotid(phosphat) (NADH, NADPH) oder von Flavinnucleotiden wie Flavinmononucleotid (FMNH) oder Flavinadenindinucleotid (FADH). Die reduzierten Nucleotide ihrerseits entstehen z.B. durch Elektronenübertragung über natürliche oder synthetische Elektronenmediatoren mit geeignetem Redoxpotential wie Ferredoxin oder Bipyridilium-Derivate, z.B. 4,4'-Bipyridilium-Derivate (Viologene) oder 2,2'-Bipyridilium-Derivate (beispielsweise Diquat-Dikationen wie Diquat-dibromid oder Diquat-dichlorid). Es sind auch finale Reduktasen bekannt, die Elektronen direkt von den Mediatoren aufnehmen können.

Besonders bevorzugt ist ein Verfahren zur Herstellung von 2-Hydroxy-4-phenylbuttersäure, bevorzugt von 2-(R)-Hydroxy-4-phenylbuttersäure, aus 2-Oxo-4-phenylbuttersäure mit Hilfe von erfindungsgemässen Biokatalysatoren, deren enzymatisch aktives Material eine Oxidoreduktase enthält oder eine Oxidoreduktase ist oder aus ganzen Zellen eines Bakteriums des Genus Proteus besteht, insbesondere der Spezies Proteus vulgaris und/oder Proteus mirabilis, bevorzugt Proteus vulgaris. 2-(R)-Hydroxy-4-phenylbuttersäure ist ein wertvolles Zwischenprodukt bei der Herstellung von ACE (angiotensin converting enzyme)-Inhibitoren oder deren Vorstufen. Diese Wirkstoffklasse hat in den vergangenen Jahren wachsendes Interesse gefunden. Sie erweitert das Potential der verfügbaren Antihypertensiva und damit die therapeutischen Möglichkeiten bei der Bekämpfung des Bluthochdrucks. Von besonderem Interesse ist dabei die Herstellung des ACE-Hemmers 3-{[1-Ethoxycarbonyl-3-phenyl-(1S)-propyl]amino}-2,3,4,5-tetrahydro-2-oxo-1H-1-(3S)-benzazepin-1-essigsäure-monohydrochlorid (= Benazepril).

Die vorliegende Erfindung betrifft insbesondere ein Verfahren zur Herstellung von 2-(R)-Hydroxy-4-phenylbuttersäure aus 2-Oxo-4-phenylbuttersäure, dadurch gekennzeichnet, dass erfindungsgemässe perlförmige Biokatalysatoren, deren enzymatisch aktives Material aus ganzen Zellen eines Bakteriums des Genus Proteus besteht, insbesondere der Spezies Proteus vulgaris und/oder Proteus mirabilis, bevorzugt Proteus vulgaris, in einem Festbett- oder Wirbelschichtreaktor, bevorzugt in einem Festbettreaktor, eingesetzt werden, durch den kontinuierlich eine wässrige Lösung des Substrats 2-Oxo-4-phenylbuttersäure, beispielsweise in einer Konzentration im Bereich von 50 bis 200 mM, von Formiat, z.B. eines Alkalimetallformiats wie Kalium- oder Natriumformiat, beispielsweise in einer Konzentration im Bereich von 100 bis 500 mM, und eines Elektronenmediators, z.B. eines 4,4'-Bipyridilium-Derivates wie Methylviologen, Carbamoylmethylviologen oder Benzylviologen, oder eines 2,2'-Bipyridilium-Derivates wie Diquat-Dikation, beispielsweise in einer Konzentration im Bereich von 0,5 bis 10 mM, geleitet wird. Die Reduktion des Substrats wird beispielsweise durch die in Proteus vulgaris enthaltene 2-Oxocarbonsäurereduktase (2-Hydroxycarboxylat-Viologen-Oxidoreduktase) katalysiert. Da dieses Enzym eine hohe Enantioselektivität aufweist und das Produkt mit einem Enantiomerenüberschuss von mehr als 99,8 % entsteht, liegt der besondere Vorteil der Verwendung des nach dem erfindungsgemässen Verfahren erhaltenen Produkts darin, dass bei der über zahlreiche Stufen verlaufenden Synthese von ACE-Hemmern bereits in einem relativ frühen Stadium der Synthese eine enantiomerenreine Verbindung eingesetzt werden kann. Das oben beschriebene Verfahren ermöglicht hohe Umsätze von mehr als 99 %.

Die nachfolgenden Beispiele sollen die Erfindung erläutern, ohne sie, etwa auf den Umfang der Beispiele, zu beschränken.

### Abkürzungen

- ee: Enantiomerenüberschuss ("enantiomeric excess")
- HPLC: high pressure liquid chromatography
- rpm: Umdrehungen ("revolutions") pro Minute

### Beispiele

### Beispiel 1: Herstellung von perlförmigen Biokatalysatoren mit Proteus vulgaris

Proteus vulgaris (ATCC 9484) wird in einem Vollmedium (Hefeextrakt 5 g/l, Glucose Monohydrat 5 g/l, K₂HPO₄ 5 g/l, Pepton aus Fleisch 20 g/l, pH 7,0) 24 h bei 37°C unter leichter Begasung mit N₂ gezüchtet. Die so erhaltenen Zellen werden bei 12°C in einer CEPA Zentrifuge Typ Z 41 G (Durchsatz 300 l/h) unter N₂-Spülung bei einer Drehzahl von 20'000 rpm (16'950 g) geerntet. Aus 400 l Kulturflüssigkeit erhält man 580 g Bakteriensediment mit einem Feuchtigkeitsgehalt von ca. 80 %. 1 Gewichtsteil Bakterienfeuchtmasse wird zusammen mit 1 Gewichtsteil Kieselsäure (Baker Produkt Nr. 254; Korngrösse ca. 20 »m, 95 % >3 »m, 95 % <60 »m; Schüttgewicht ca. 50 g/100 ml) in 3 Gewichtsteilen 50 mM Natriumacetatpuffer pH 5,0 aufgeschlämmt und anschliessend mit 5 Gewichtsteilen einer 1,4%igen (w/w) hochviskosen wässrigen Chitosan-Acetat-Lösung (Viskosität ca. 10'000 cP bei 20°C und 10 rpm; pH 4,5 mit Essigsäure eingestellt; unlösliche Bestandteile abgesiebt) unter kräftigem Rühren gemischt. Diese Suspension (Viskosität ca. 2'000 cP) wird unter leichtem Rühren tropfenweise zu einer 1,5%igen (w/v) Natriumtripolyphosphatlösung (mindestens dreifaches Suspensionsvolumen; pH 8,1 mit Phosphorsäure eingestellt) gegeben. Dazu verwendet man eine Spritze mit einer Kanülenöffnung vom Durchmesser 1,4 mm und mit einem Durchsatz von 1 ml/min. Die Tropfen werden mit komprimiertem Stickstoff von der Kanüle abgeblasen (Fallweg 10-15 cm). Es entstehen perlförmige Teilchen mit einem Durchmesser von 2-3 mm, die zur Schrumpfung und Härtung 2 Stunden in dem Natriumtripolyphosphat-Vernetzerbad verbleiben. Man erhält aus 100 g Suspension ca. 40 g Perlen (Schüttvolumen ca. 80 ml) mit einer Zellkonzentration von 0,25 g Bakterienfeuchtmasse bzw. von 0,05 g Trockenzellen pro g Perlen, die im folgenden mit Bezug auf die Konzentration der Kieselsäure in der gespritzten Suspension als "10%ige Kieselsäure-Biokatalysatorperlen" bezeichnet werden.

Zum Vergleich werden "Kontrolle-Biokatalysatorperlen" ohne den Zusatz von Kieselsäure hergestellt. Dazu wird 1 Gewichtsteil Bakterienfeuchtmasse in 4 Gewichtsteilen Natriumacetatpuffer pH 5,0 aufgeschlämmt und dann mit 5 Gewichtsteilen der oben beschriebenen Chitosan-Acetat-Lösung gemischt. Die Suspension wird zur Herstellung von perlförmigen Teilchen wie im vorangegangenen angegeben in Natriumtripolyphosphatlösung getropft.

Bei der Herstellung der "Kontrolle-Biokatalysatorperlen" kommt es beim Aufschlämmen zu erheblicher Schaumbildung, die bei der Herstellung der "10%ige Kieselsäure-Biokatalysatorperlen" unterbleibt, da die Kieselsäure als Antischaummittel wirkt. Durch die Schaumbildung sind die "Kontrolle-Biokatalysatorperlen" leichter als die Perlen mit Kieselsäurezusatz und haben die Tendenz, im Vernetzerbad an die Oberfläche zu steigen.

### Beispiel 2: Bestimmung der Druckfestigkeit der Biokatalvsatorperlen

Die Druckfestigkeit der Biokatalysatorperlen wird nach einem Tag Lagerung bei 4°C in der Natriumtripolyphosphatlösung pH 8,1 mit einem Messsystem für thermomechanische Analysen (TA 3000-System mit Messzelle TMA 40, Mettler Instrumente AG, Greifensee, Schweiz) bestimmt. Jeweils drei Biokatalysatorperlen (Durchmesser 3 mm) in feuchtem Zustand werden in Dreiecksanordnung angrenzend auf den Probentisch gelegt und zentrisch mit einer mit Natriumtripolyphosphatlösung getränkten Keramikscheibe (Durchmesser 6 mm, Dicke 0,7 mm, Trockengewicht 70 mg) bedeckt, auf die der Messfühler positioniert wird. Die Deformation der Probe (Abnahme der Dicke der Perlen in »m) wird durch Niederdrücken der Keramikscheibe unter einer Belastung von 0,02 N isotherm bei 30°C in Funktion der Zeit gemessen. Die Ergebnisse sind in Tabelle 1 zusammengestellt.

**Tabelle 1**

| Bestimmung der Druckfestigkeit der Biokatalysatorperlen | | |
|---|---|---|
| | Deformation der 3 mm-Biokatalysatorperlen [»m] | |
| Zeit [min] | Kontrolle-Biokatalysatorperlen | 10%ige Kieselsäure-Biokatalysatorperlen |
| 0,5 | 40 | 10 |
| 1 | 80 | 20 |
| 2 | 130 | 30 |
| 4 | 200 | 40 |

Tabelle 1 macht deutlich, dass die "10%ige Kieselsäure-Biokatalysatorperlen" eine wesentlich höhere Druckfestigkeit aufweisen als die "Kontrolle-Biokatalysatorperlen". Nach 15 Minuten zeigen die "10%ige Kieselsäure-Biokatalysatorperlen" fast keine weitere Deformationszunahme mehr.

### Beispiel 3: Herstellung von 2-(R)-Hydroxy-4-phenylbuttersäure mit Hilfe von perlförmigen Biokatalysatoren im Labormassstab

48 ml (Schüttvolumen) "10%ige Kieselsäure-Biokatalysatorperlen", die aus 60 g Suspension gemäss Beispiel 1 hergestellt werden, werden vom Natriumtripolyphosphat-Vernetzerbad getrennt und zu 200 ml einer mit Stickstoff vorbegasten Lösung von 50 mM (1,84 % w/v) Natriumtripolyphosphatpuffer pH 7,0, 100 mM Kaliumformiat und 1 mM Carbamoylmethylviologen (1,1'-Dicarbamoylmethyl-4,4'-dipyridiniumdikation) zugegeben. In dieser Reduzierlösung verbleiben die Perlen unter Vakuum zur Entgasung bis zur vollständigen Violettfärbung, die auf der Reduktion des Carbamoylmethylviologens durch die in Proteus vulgaris enthaltene Formiatdehydrogenase beruht. Anschliessend werden die Perlen in einen Enzymfestbettreaktor gegeben, i.e. in eine mit einem Mantel versehene Glassäule (Innendurchmesser 1,6 cm, Betthöhe 24 cm, 25°C). Die mit Stickstoff vorbegaste Substratlösung (50 mM Natriumtripolyphosphatpuffer pH 6,7, 100 mM 2-Oxo-4-phenylbuttersäure, 300 mM Kaliumformiat, 3 mM Carbamoylmethylviologen) wird mittels einer Dosierpumpe bei 2,0-3,0 bar Ueberdruck mit einem Durchsatz von zunächst 120 ml/h, dann mit 24 ml/h kontinuierlich durch die Säule geleitet. Der Umsatz der Reduktion der 2-Oxo-4-phenylbuttersäure zur 2-(R)-Hydroxy-4-phenylbuttersäure, die durch die in Proteus vulgaris enthaltene 2-Oxocarbonsäurereduktase (2-Hydroxycarboxylat-Viologen-Oxidoreduktase) katalysiert wird, wird durch HPLC-Analyse gemessen (Säule: Nucleosil C18, Partikelgrösse 5 »m, Länge 12,5 cm, Innendurchmesser 4,6 mm; Durchsatz 1,0 ml/min.; Laufmittel: 3 Volumenteile Acetonitril/8 Volumenteile 100 mM Kaliumphosphatpuffer pH 3,0).

In analoger Weise werden die "Kontrolle-Biokatalysatorperlen" (Schüttvolumen 49 ml aus 60 g Suspension gemäss Beispiel 1) zur Reduktion von 2-Oxo-4-phenylbuttersäure eingesetzt. Dabei ist es notwendig, das Perlenbett mit dem Säulenadapter einzuschränken, weil die Perlen zum überwiegenden Teil aufschwimmen und sich daher Leerzonen in der Säule ergeben.

Die Ergebnisse der Produktivitätsbestimmung sind in Tabelle 2 zusammengefasst.

**Tabelle 2**

| Herstellung von 2-(R)-Hydroxy-4-phenylbuttersäure im Festbettreaktor mit Hilfe von perlförmigen Biokatalysatoren im Labormassstab (Umsatz) | | |
|---|---|---|
| | Umsatz im Festbettreaktor bei 25°C | |
| Durchsatz [ml/h] | Kontrolle-Biokatalysatorperlen | 10%ige Kieselsäure Biokatalysatorperlen |
| 120 | 60 % | 70 % |
| 24 | 96 % | >99,5 % |

Aus Tabelle 2 wird ersichtlich, dass die "Kontrolle-Biokatalysatorperlen" eine deutlich schlechtere Produktivität aufweisen als die "10%ige Kieselsäure-Biokatalysatorperlen".

Aufgrund der zunehmenden Kompaktierung der Perlen ist ein Dauerbetrieb des Kontroll-Enzymreaktors nicht möglich, da die Säule bereits nach einem Tag bei einem Durchsatz von 24 ml/h verstopft ist. Mit den "10%ige Kieselsäure-Biokatalysatorperlen" dagegen ist der Dauerbetrieb unter den gleichen Bedingungen problemlos.

### Beispiel 4: Herstellung von 2-(R)-Hydroxy-4-phenylbuttersäure mit Hilfe von perlförmigen Biokatalysatoren im Pilotmassstab und Isolierung des Produkts

Die Herstellung von "10%ige Kieselsäure-Biokatalysatorperlen" erfolgt aus 573 g Bakteriensediment gemäss Beispiel 1, wobei jedoch anstatt der Spritze eine Einrichtung bestehend aus einer Zahnradpumpe (Durchsatz 1,4 l/h; Druck ca. 0,5 bar) und einer 7-strahligen Dusche mit 0,6 mm Kapillaren zur Tropfenbildung eingesetzt wird. Zur Vergrösserung der Abtropffrequenz bzw. zur Beeinflussung der Tropfengrösse besitzt jede Kapillare einen separaten Luftausströmkanal. Nach Schrumpfung und Härtung in einer 3%igen (w/v) Natriumtripolyphosphatlösung erhält man aus 573 g Bakteriensediment ca. 4 l (Schüttvolumen) Perlen.

2-(R)-Hydroxy-4-phenylbuttersäure wird analog zu der in Beispiel 3 angegebenen Weise hergestellt. Die 4 l (Schüttvolumen) "10%ige Kieselsäure-Biokatalysatorperlen" werden vom Natriumtripolyphosphat-Vernetzerbad getrennt und zu 6 l einer mit Stickstoff vorbegasten Lösung von 100 mM (3,68 % w/v) Natriumtripolyphosphat pH 7,0, 100 mM Kaliumformiat und 1 mM Carbamoylmethylviologen gegeben. In dieser Reduzierlösung verbleiben die Perlen unter Vakuum zur Entgasung bis zur vollständigen Violettfärbung. Anschliessend werden die Perlen in eine Chromatographie-Säule (Innendurchmesser 11,3 cm, Grundfläche 100 cm², Länge 60 cm, Betthöhe 40 cm) gegeben. Die mit Stickstoff vorbegaste Substratlösung (100 mM Natriumtripolyphosphatpuffer pH 6,7, 112,2 mM 2-Oxo-4-phenylbuttersäure, 300 mM Kaliumformiat, 1 mM Carbamoylmethylviologen) wird sterilfiltriert und mittels einer Dosierpumpe bei 2,0-3,0 bar Ueberdruck mit einem Durchsatz von zunächst 2,5 l/h kontinuierlich durch die Säule geleitet. Der durch HPLC gemessene Umsatz ist > 99,5 %. Die Säule wird bei Raumtemperatur, d.h. bei etwa 23 bis 26,5°C, Tag und Nacht ohne Unterbruch betrieben.

Der Umsatz wird durch tägliche HPLC-Analysen kontrolliert und durch Regulation der Pumpgeschwindigkeit bei Umsatzwerten von > 99,5 % gehalten. Nach 25 Tagen sind insgesamt ca. 800 l Reaktionslösung umgesetzt (durchschnittlicher Umsatz 99,6 %) und der Durchsatz beträgt noch 1 l/h.

1160 l umgesetzte Reaktionslösung werden mit 250 l Essigester gemischt und mit 114 l 42,5%iger ortho-Phosphorsäure auf pH 2,6 gestellt. Die 2-(R)-Hydroxy-4-phenylbuttersäure wird mit einer Ausbeute von 94 % in Essigester extrahiert. Die in der Wasserphase verbliebene 2-(R)-Hydroxy-4-phenylbuttersäure wird noch zweimal mit je 120 l Essigester weiterextrahiert. Nach den drei Extraktionsstufen ist die Extraktion der 2-(R)-Hydroxy-4-phenylbuttersäure vollständig (99,8 %). Nach Vereinigung der drei Essigesterphasen (Gesamtvolumen 450 l), Zugabe von 250 l Essigester und Abdestillieren von 470 l des Lösungsmittels wird die Lösung durch einen Einplattenfilter klarfiltriert. 50 l Essigester werden für das Spülen des Filters verwendet und zu der klaren Lösung gegeben. Nach Abdestillieren von 200 l des Lösungsmittels beträgt das Endvolumen der Lösung 80 l. Zu dieser Lösung werden 400 l Cyclohexan gegeben und 200 l des Lösungsmittels werden abdestilliert. Nach Zentrifugation des auskristallisierten Produkts und Trocknen im Vakuum bei 20-25°C bis zur Gewichtskonstanz werden 23,4 kg kristalline 2-(R)-Hydroxy-4-phenylbuttersäure erhalten.

Zur Kontrolle der Enantiomerenreinheit wird eine Probe der kristallinen Säure in absolutem Ethanol gelöst und mit Chlorwasserstoffgas während 24 Stunden bei Raumtemperatur zur Reaktion gebracht. Nach Abdestillieren des Alkohols und kurzem Entgasen im Hochvakuum verbleibt ein hellgelbes Oel, das an einer chiralen Säule (250 x 4,6 mm i.D., Durchfluss 1 ml/min, stationäre Phase Chiralcel OD [Stehelin, Basel] Typ OD-5-15-20925, mobile Phase: 90 % Hexan - 10 % Isopropanol - 0,1 % Diethylamin) durch HPLC bei 25°C/32 bar analysiert wird. Die zu analysierenden Substanzen liegen in einer Konzentration von 1 mg/ml im Fliessmittel vor (Einspritzmenge 10 »l). Das Scanning erfolgt bei einer Wellenlänge von 210 nm, die Auswertung durch Flächenvergleich mit externem Standard. Der ermittelte ee-Wert ist >99,8 %.

## Patentansprüche (Patentansprüche für folgende(n) Vertragsstaat(en): AT, BE, CH, DE, FR, GB, IT, LI, LU, NL, SE)

1. Ein perlförmiger Biokatalysator mit hoher mechanischer Festigkeit umfassend enzymatisch aktives Material, einen kationischen Polyelektrolyten und mehrwertige Anionen, dadurch gekennzeichnet, dass der Biokatalysator Kieselsäure enthält und erhältlich ist aus einer Suspension aus dem enzymatisch aktiven Material der Kieselsäure, Pufferlösung und dem Polyelektrolyten, worin die Endkonzentration von Kieselsäure in einem Bereich zwischen 2 bis 15 % (w/w) liegt.

2. Ein perlförmiger Biokatalysator gemäss Anspruch 1, dadurch gekennzeichnet, dass das enzymatisch aktive Material aus ganzen Zellen, Zellfragmenten oder Zellorganellen eines Mikroorganismus, aus Enzymen oder aus einer beliebigen Kombination dieser Arten des enzymatisch aktiven Materials besteht.

3. Ein perlförmiger Biokatalysator gemäss Anspruch 1 oder 2, dadurch gekennzeichnet, dass das enzymatisch aktive Material aus ganzen Zellen eines Bakteriums oder einer Hefe besteht.

4. Ein perlförmiger Biokatalysator gemäss Anspruch 3, dadurch gekennzeichnet, dass das Bakterium zum Genus Proteus gehört.

5. Ein perlförmiger Biokatalysator gemäss Anspruch 4, dadurch gekennzeichnet, dass das Bakterium zur Spezies Proteus vulgaris und/oder zur Spezies Proteus mirahilis gehört.

6. Ein perlförmiger Biokatalysator gemäss Anspruch 1 oder 2, dadurch gekennzeichnet, dass das enzymatisch aktive Material eine Oxidoreduktase enthält oder eine Oxidoreduktase ist.

7. Ein perlförmiger Biokatalysator gemäss einem der Ansprüche 1 bis 6, dadurch gekennzeichnet, dass der kationische Polyelektrolyt Chitosan ist.

8. Ein perlförmiger Biokatalysator gemäss Anspruch 7, dadurch gekennzeichnet, dass das Chitosan ein Molekulargewicht von etwa 50'000 bis etwa 3'000'000 hat.

9. Ein perlförmiger Biokatalysator gemäss einem der Ansprüche 1 bis 8, dadurch gekennzeichnet, dass die mehrwertigen Anionen mehrwertige anorganische Anionen sind.

10. Ein perlförmiger Biokatalysator gemäss Anspruch 9, dadurch gekennzeichnet, dass die anorganischen Anionen Orthophosphat, Metaphosphate, Pyrophosphate, Polyphosphate oder Cyanoferrate sind.

11. Ein perlförmiger Biokatalysator gemäss Anspruch 10, dadurch gekennzeichnet, dass die anorganischen Anionen Tripolyphosphat sind.

12. Ein perlförmiger Biokatalysator gemäss einem der Ansprüche 1 bis 8, dadurch gekennzeichnet, dass die mehrwertigen Anionen mehrwertige organische Anionen sind.

13. Ein perlförmiger Biokatalysator gemäss Anspruch 12, dadurch gekennzeichnet, dass die organischen Anionen polymere organische Carboxylate, Sulfonate oder Hydroxyverbindungen sind.

14. Ein perlförmiger Biokatalysator gemäss einem der Ansprüche 1 bis 13, dadurch gekennzeichnet, dass die Kieselsäure eine gefällte Kieselsäure ist.

15. Ein perlförmiger Biokatalysator gemäss Anspruch 14, dadurch gekennzeichnet, dass die gefällte Kieselsäure eine durchschnittliche Korngrösse im Bereich von 5 bis 50 »m und ein durchschnittliches Schüttgewicht im Bereich von 5 bis 100 g/100 ml hat.

16. Verfahren zur Herstellung von perlförmigen Biokatalysatoren gemäss einem der Ansprüche 1 bis 15, dadurch gekennzeichnet, dass enzymatisch aktives Material mit gefällter Kieselsäure in fester Form, einer wässrigen Pufferlösung und der wässrigen Lösung eines kationischen Polyelektrolyten unter Vermeidung von Schaumbildung zu einer Suspension vermischt wird, die Suspension in ein wässriges Vernetzerbad enthaltend mehrwertige Anionen tropfenweise eingebracht wird und die entstandenen Biokatalysatorperlen im Vernetzerbad geschrumpft und verfestigt und gegebenenfalls vom Vernetzerbad getrennt werden.

17. Verfahren gemäss Anspruch 16, dadurch gekennzeichnet, dass die Schaumbildung vermieden wird, indem die Vermischung in Gegenwart der Kieselsäure erfolgt.

18. Verfahren gemäss Anspruch 16 oder 17, dadurch gekennzeichnet, dass als kationischer Polyelektrolyt Chitosan in wässriger Lösung mit einer Viskosität im Bereich von 1'000 bis 20'000 cP eingesetzt wird.

19. Verfahren gemäss einem der Ansprüche 16 bis 18, dadurch gekennzeichnet, dass das Verhältnis der Gewichtsanteile von Kieselsäure zu kationischem Polyelektrolyt in einem Bereich von 1,5 bis 50 zu 1 liegt.

20. Verfahren gemäss einem der Ansprüche 16 bis 19, dadurch gekennzeichnet, dass die Endkonzentration in der Suspension aus enzymatisch aktivem Material, Kieselsäure, Pufferlösung und kationischem Polyelektrolyt für den kationischen Polyelektrolyt in einem Bereich von 0,3 bis 1,5 % (w/w) liegt.

21. Verfahren gemäss einem der Ansprüche 16 bis 20, dadurch gekennzeichnet, dass das mehrwertige Anion im Vernetzerbad Tripolyphosphat ist.

22. Verfahren gemäss Anspruch 21, dadurch gekennzeichnet, dass Tripolyphosphorsäure oder ein Salz davon in einer Konzentration im Bereich von 0,5 bis 10 % (w/v) für das Vernetzerbad eingesetzt wird.

23. Verfahren gemäss Anspruch 21 oder 22, dadurch gekennzeichnet, dass das Volumen des Vernetzerbades mindestens etwa das dreifache Volumen der Suspension aus enzymatisch aktivem Material, Kieselsäure, Pufferlösung und kationischem Polyelektrolyt beträgt.

24. Perlförmige Biokatalysatoren mit hoher mechanischer Festigkeit, dadurch gekennzeichnet, dass sie nach einem Verfahren gemäss einem der Ansprüche 16 bis 23 hergestellt werden.

25. Verfahren zur Umsetzung organischer Substanzen, dadurch gekennzeichnet, dass für die Umsetzung ein perlförmiger Biokatalysator gemäss einem der Ansprüche 1 bis 15 eingesetzt wird.

26. Verfahren zur Herstellung von α-Hydroxycarbonsäuren aus α-Ketocarbonsauren, dadurch gekennzeichnet, dass für die Umsetzung ein perlförmiger Biokatalysator gemäss einem der Ansprüche 4 bis 6 eingesetzt wird.

27. Verfahren gemäss Anspruch 26 zur Herstellung von 2-Hydroxy-4-phenylbuttersäure aus 2-Oxo-4-phenylbuttersaure.

28. Verfahren gemäss Anspruch 26 oder 27 zur Herstellung von 2-(R)-Hydroxy-4-phenylbuttersäure aus 2-Oxo-4-phenylbuttersäure, dadurch gekennzeichnet, dass die perlförmigen Biokatalysatoren in einem Festbettreaktor eingesetzt werden, durch den kontinuierlich eine wässrige Lösung des Substrats 2-Oxo-4-phenylbuttersäure, von Formiat und eines Elektronenmediators geleitet wird.

## Patentansprüche (Patentansprüche für folgende(n) Vertragsstaat(en): ES, GR)

1. Verfahren zur Herstellung eines perlförmigen Biokatalysators mit hoher mechanischer Festigkeit umfassend enzymatisch aktives Material, einen kationischen Polyelektrolyten, mehrwertige Anionen und Kieselsäure, dadurch gekennzeichnet, dass enzymatisch aktives Material mit gefällter Kieselsäure in fester Form, einer wässrigen Pufferlösung und der wässrigen Lösung eines kationischen Polyelektrolyten unter Vermeidung von Schaumbildung zu einer Suspension, in der die Endkonzentration an Kieselsäure in einem Bereich zwischen 2 und 15 % (w/w) liegt, vermischt wird, die Suspension in ein wässriges Vernetzerbad enthaltend mehrwertige Anionen tropfenweise eingebracht wird und die entstandenen Biokatalysatorperlen im Vernetzerbad geschrumpft und verfestigt und gegebenenfalls vom Vernetzerbad getrennt werden.

2. Verfahren gemäss Anspruch 1, dadurch gekennzeichnet, dass die Schaumbildung vermieden wird, indem die Vermischung in Gegenwart der Kieselsaure erfolgt.

3. Verfahren gemäss Anspruch 1 oder 2, dadurch gekennzeichnet, dass als kationischer Polyelektrolyt Chitosan in wässriger Lösung mit einer Viskosität im Bereich von 1'000 bis 20'000 cP eingesetzt wird.

4. Verfahren gemäss einem der Ansprüche 1 bis 3, dadurch gekennzeichnet, dass das Verhältnis der Gewichtsanteile von Kieselsäure zu kationischem Polyelektrolyt in einem Bereich von 1,5 bis 50 zu 1 liegt.

5. Verfahren gemäss einem der Ansprüche 1 bis 4, dadurch gekennzeichnet, dass die Endkonzentration in der Suspension aus enzymatisch aktivem Material, Kieselsäure, Pufferlösung und kationischem Polyelektrolyt für den kationischen Polyelektrolyt in einem Bereich von 0,3 bis 1,5 % (w/w) liegt.

6. Verfahren gemäss einem der Ansprüche 1 bis 5, dadurch gekennzeichnet, dass das mehrwertige Anion im Vernetzerbad Tripolyphosphat ist.

7. Verfahren gemäss Anspruch 6, dadurch gekennzeichnet, dass Tripolyphosphorsäure oder ein Salz davon in einer Konzentration im Bereich von 0,5 bis 10 % (w/v) für das Vernetzerbad eingesetzt wird

8. Verfahren gemäss Anspruch 6 oder 7, dadurch gekennzeichnet, dass das Volumen des Vernetzerbades mindestens etwa das dreifache Volumen der Suspension aus enzymatisch aktivem Material, Kieselsäure, Pufferlösung und kationischem Polyelektrolyt beträgt.

9. Verfahren gemäss Anspruch 1 zur Herstellung eines perlförmigen Biokatalysators, dadurch gekennzeichnet, dass das enzymatisch aktive Material des Biokatalysators aus ganzen Zellen, Zellfragmenten oder Zellorganellen eines Mikroorganismus, aus Enzymen oder aus einer beliebigen Kombination dieser Arten des enzymatisch aktiven Materials besteht.

10. Verfahren gemäss Anspruch 1 zur Herstellung eines perlförmigen Biokatalysators, dadurch gekennzeichnet, dass das enzymatisch aktive Material des Biokatalysators aus ganzen Zellen eines Bakte riums oder einer Hefe besteht.

11. Verfahren gemäss Anspruch 10 zur Herstellung eines perlförmigen Biokatalysators, dadurch gekennzeichnet, dass das Bakterium zum Genus Proteus gehört.

12. Verfahren gemäss Anspruch 11 zur Herstellung eines perlförmigen Biokatalysators, dadurch gekennzeichnet, dass das Bakterium zur Spezies Proteus vulgaris und/oder zur Spezies Proteus mirabilis gehört.

13. Verfahren gemäss Anspruch 1 zur Herstellung eines perlförmigen Biokatalysators, dadurch gekennzeichnet, dass das enzymatisch aktive Material des Biokatalysators eine Oxidoreduktase enthält oder eine Oxidoreduktase ist.

14. Verfahren gemäss Anspruch 1 zur Herstellung eines perlförmigen Biokatalysators, dadurch gekennzeichnet, dass der Biokatalysator als kationischen Polyelektrolyten Chitosan enthält.

15. Verfahren gemäss Anspruch 14 zur Herstellung eines perlförmigen Biokatalysators, dadurch gekennzeichnet, dass das Chitosan ein Molekulargewicht von etwa 50'000 bis etwa 3'000'000 hat.

16. Verfahren gemäss Anspruch 1 zur Herstellung eines perlförmigen Biokatalysators, dadurch gekennzeichnet, dass der Biokatalysator als mehrwertige Anionen mehrwertige anorganische Anionen enthält.

17. Verfahren gemäss Anspruch 16 zur Herstellung eines perlförmigen Biokatalysators, dadurch gekennzeichnet, dass die anorganischen Anionen Orthophosphat, Metaphosphate, Pyrophosphate, Polyphosphate oder Cyanoferrate sind.

18. Verfahren gemäss Anspruch 17 zur Herstellung eines perlförmigen Biokatalysators, dadurch gekennzeichnet, dass die anorganischen Anionen Tripolyphosphat sind.

19. Verfahren gemäss Anspruch 1 zur Herstellung eines perlförmigen Biokatalysators, dadurch gekennzeichnet, dass der Biokatalysator als mehrwertige Anionen mehrwertige organische Anionen enthält.

20. Verfahren gemäss Anspruch 19 zur Herstellung eines perlförmigen Biokatalysators, dadurch gekennzeichnet, dass die organischen Anionen polymere organische Carboxylate, Sulfonate oder Hydroxyverbindungen sind.

21. Verfahren gemäss Anspruch 1 zur Herstellung eines perlförmigen Biokatalysators, dadurch gekennzeichnet, dass die Kieselsäure des Biokatalysators eine gefällte Kieselsäure ist.

22. Verfahren gemäss Anspruch 21 zur Herstellung eines perlförmigen Biokatalysators, dadurch gekennzeichnet, dass die gefällte Kieselsäure eine durchschnittliche Korngrösse im Bereich von 5 bis 50 »m und ein durchschnittliches Schüttgewicht im Bereich von 5 bis 100 g/100 ml hat.

23. Verfahren zur Umsetzung organischer Substanzen, dadurch gekennzeichnet, dass für die Umsetzung ein perlförmiger Biokatalysator eingesetzt wird, der nach einem Verfahren gemäss der Ansprüche 1 bis 22 hergestellt wurde.

24. Verfahren zur Herstellung von α-Hydroxycarbonsäuren aus α-Ketocarbonsäuren, dadurch gekennzeichnet, dass für die Umsetzung ein perlförmiger Biokatalysator eingesetzt wird, der nach einem Verfahren gemäss der Ansprüche 11 bis 13 hergestellt wurde.

25. Verfahren gemäss Anspruch 24 zur Herstellung von 2-Hydroxy-4-phenylbuttersäure aus 2-Oxo-4-phenylbuttersäure.

26. Verfahren gemäss Anspruch 24 oder 25 zur Herstellung von 2-(R)-Hydroxy-4-phenylbuttersäure aus 2-Oxo-4-phenylbuttersäure, dadurch gekennzeichnet, dass die perlförmigen Biokatalysatoren in einem Festbettreaktor eingesetzt werden, durch den kontinuierlich eine wässrige Lösung des Substrats 2-Oxo-4-phenylbuttersäure, von Formiat und eines Elektronenmediators geleitet wird.

## Claims (Claims for the following Contracting State(s): AT, BE, CH, DE, FR, GB, IT, LI, LU, NL, SE)

1. A spherical biocatalyst of high mechanical strength, comprising enzymatically active material, a cationic polyelectrolyte and polyvalent anions, wherein the biocatalyst comprises silicic acid and is obtainable from a suspension of the enzymatically active material, the silicic acid, a buffer solution and the polyelectrolyte, in which suspension the final concentration of silicic acid is in a range of from 2 to 15 % (w/w).

2. A spherical biocatalyst according to claim 1, wherein the enzymatically active material consists of whole cells, cell fragments or cell organelles of a microorganism, or of enzymes or of any combination of these types of enzymatically active material.

3. A spherical biocatalyst according to either claim 1 or claim 2, wherein the enzymatically active material consists of whole cells of a bacterium or yeast.

4. A spherical biocatalyst according to claim 3, wherein the bacterium belongs to the genus Proteus.

5. A spherical biocatalyst according to claim 4, wherein the bacterium belongs to the species Proteus vulgaris and/or the species Proteus mirabilis.

6. A spherical biocatalyst according to either claim 1 or claim 2, wherein the enzymatically active material comprises an oxido-reductase or is an oxido-reductase.

7. A spherical biocatalyst according to any one of claims 1 to 6, wherein the cationic polyelectrolyte is chitosan.

8. A spherical biocatalyst according to claim 7, wherein the chitosan has a molecular weight of from approximately 50 000 to approximately 3 000 000.

9. A spherical biocatalyst according to any one of claims 1 to 8, wherein the polyvalent anions are polyvalent inorganic anions.

10. A spherical biocatalyst according to claim 9, wherein the inorganic anions are orthophosphate, metaphosphates, pyrophosphates, polyphosphates or cyanoferrates.

11. A spherical biocatalyst according to claim 10, wherein the inorganic anions are tripolyphosphate.

12. A spherical biocatalyst according to any one of claims 1 to 8, wherein the polyvalent anions are polyvalent organic anions.

13. A spherical biocatalyst according to claim 12, wherein the organic anions are polymeric organic carboxylates, sulfonates or hydroxy compounds.

14. A spherical biocatalyst according to any one of claims 1 to 13, wherein the silicic acid is a precipitated silicic acid.

15. A spherical biocatalyst according to claim 14, wherein the precipitated silicic acid has an average particle size in the range of from 5 to 50 »m and an average bulk density in the range of from 5 to 100 g/100 ml.

16. A process for the manufacture of spherical biocatalysts according to any one of claims 1 to 15, which comprises mixing enzymatically active material with precipitated silicic acid in solid form, an aqueous buffer solution and an aqueous solution of a cationic polyelectrolyte to form a suspension, while avoiding the formation of foam, introducing the suspension dropwise into an aqueous crosslinker bath comprising polyvalent anions, and shrinking and solidifying the resulting biocatalyst beads in the crosslinker bath, and optionally separating them from the crosslinker bath.

17. A process according to claim 16, which comprises avoiding the formation of foam by carrying out the mixing process in the presence of silicic acid.

18. A process according to either claim 16 or claim 17, wherein chitosan in aqueous solution having a viscosity in the range of from 1000 to 20 000 cP is used as cationic polyelectrolyte.

19. A process according to any one of claims 16 to 18, wherein the ratio by weight of silicic acid to cationic polyelectrolyte is in a range of from 1.5 to 50 : 1.

20. A process according to any one of claims 16 to 19, wherein the final concentration of the cationic polyelectrolyte in the suspension of enzymatically active material, silicic acid, buffer solution and cationic polyelectrolyte is in a range of from 0.3 to 1.5 % (w/w).

21. A process according to any one of claims 16 to 20, wherein the polyvalent anion in the crosslinker bath is tripolyphosphate.

22. A process according to claim 21, wherein tripolyphosphoric acid or a salt thereof is used for the crosslinker bath in a concentration in the range of from 0.5 to 10 % (w/v).

23. A process according to either claim 21 or claim 22, wherein the volume of the crosslinker bath is at least about three times the volume of the suspension of enzymatically active material, silicic acid, buffer solution and cationic polyelectrolyte.

24. Spherical biocatalysts of high mechanical strength manufactured by a process according to any one of claims 16 to 23.

25. A process for the conversion of organic substances, wherein a spherical biocatalyst according to any one of claims 1 to 15 is used for the conversion.

26. A process for the manufacture of α-hydroxycarboxylic acids from α-ketocarboxylic acids, wherein a spherical biocatalyst according to any one of claims 4 to 6 is used for the conversion.

27. A process according to claim 26 for the manufacture of a 2-hydroxy-4-phenylbutyric acid from 2-oxo-4-phenylbutyric acid.

28. A process according to either claim 26 or claim 27 for the manufacture of 2-(R)-hydroxy-4-phenylbutyric acid from 2-oxo-4-phenylbutyric acid, wherein the spherical biocatalysts are employed in a fixed-bed reactor through which there is continuously passed an aqueous solution of the substrate 2-oxo-4-phenylbutyric acid, formate and an electron mediator.

## Claims (Claims for the following Contracting State(s): ES, GR)

1. A process for the manufacture of a spherical biocatalyst of high mechanical strength, comprising enzymatically active material, a cationic polyelectrolyte, polyvalent anions and silicic acid, which process comprises mixing ensymatically active material with precipitated silicic acid in solid form, an aqueous buffer solution and an aqueous solution of a cationic polyelectrolyte to form a suspension in which the final concentration of silicic acid is in a range of from 2 to 15 % (w/w), while avoiding the formation of foam, introducing the suspension dropwise into an aqueous crosslinker bath comprising polyvalent anions, and shrinking and solidifying the resulting biocatalyst beads in the crosslinker bath, and optionally separating them from the crosslinker bath.

2. A process according to claim 1, which comprises avoiding the formation of foam by carrying out the mixing process in the presence of silicic acid.

3. A process according to either claim 1 or claim 2, wherein chitosan in aqueous solution having a viscosity in the range of from 1000 to 20 000 cP is used as cationic polyelectrolyte.

4. A process according to any one of claims 1 to 3, wherein the ratio by weight of silicic acid to cationic polyelectrolyte is in a range of from 1.5 to 50 : 1.

5. A process according to any one of claims 1 to 4, wherein the final concentration of the cationic polyelectrolyte in the suspension of enzymatically active material, silicic acid, buffer solution and cationic polyelectrolyte is in a range of from 0.3 to 1.5 % (w/w).

6. A process according to any one of claims 1 to 5, wherein the polyvalent anion in the crosslinker bath is tripolyphosphate.

7. A process according to claim 6, wherein tripolyphosphoric acid or a salt thereof is used for the crosslinker bath in a concentration in the range of from 0.5 to 10 % (w/v).

8. A process according to either claim 6 or claim 7, wherein the volume of the crosslinker bath is at least about three times the volume of the suspension of enzymatically active material, silicic acid, buffer solution and cationic polyelectrolyte.

9. A process according to claim 1 for the manufacture of a spherical biocatalyst, wherein the enzymatically active material of the biocatalyst consists of whole cells, cell fragments or cell organelles of a microorganism, or of enzymes or of any combination of these types of enzymatically active material.

10. A process according to claim 1 for the manufacture of a spherical biocatalyst, wherein the enzymatically active material of the biocatalyst consists of whole cells of a bacterium or yeast.

11. A process according to claim 10 for the manufacture of a spherical biocatalyst, wherein the bacterium belongs to the genus Proteus.

12. A process according to claim 11 for the manufacture of a spherical biocatalyst, wherein the bacterium belongs to the species Proteus vulgaris and/or the species Proteus mirabilis.

13. A process according to claim 1 for the manufacture of a spherical biocatalyst, wherein the enzymatically active material of the biocatalyst comprises an oxido-reductase or is an oxido-reductase.

14. A process according to claim 1 for the manufacture of a spherical biocatalyst, wherein the biocatalyst comprises chitosan as cationic polyelectrolyte.

15. A process according to claim 14 for the manufacture of a spherical biocatalyst, wherein the chitosan has a molecular weight of from approximately 50 000 to approximately 3 000 000.

16. A process according to claim 1 for the manufacture of a spherical biocatalyst, wherein the biocatalyst comprises polyvalent inorganic anions as polyvalent anions.

17. A process according to claim 16 for the manufacture of a spherical biocatalyst, wherein the inorganic anions are orthophosphate, metaphosphates, pyrophosphates, polyphosphates or cyanoferrates.

18. A process according to claim 17 for the manufacture of a spherical biocatalyst, wherein the inorganic anions are tripolyphosphate.

19. A process according to claim 1 for the manufacture of a spherical biocatalyst, wherein the biocatalyst comprises polyvalent organic anions as polyvalent anions.

20. A process according to claim 19 for the manufacture of a spherical biocatalyst, wherein the organic anions are polymeric organic carboxylates, sulfonates or hydroxy compounds.

21. A process according to claim 1 for the manufacture of a spherical biocatalyst, wherein the silicic acid of the biocatalyst is a precipitated silicic acid.

22. A process according to claim 21 for the manufacture of a spherical biocatalyst, wherein the precipitated silicic acid has an average particle size in the range of from 5 to 50 »m and an average bulk density in the range of from 5 to 100 g/100 ml.

23. A process for the conversion of organic substances, wherein a spherical biocatalyst prepared by a process according to any one of claims 1 to 22 is used for the conversion.

24. A process for the manufacture of α-hydroxycarboxylic acids from α-ketocarboxylic acids, wherein a spherical biocatalyst prepared by a process according to any one of claims 11 to 13 is used for the conversion.

25. A process according to claim 24 for the manufacture of 2-hydroxy-4-phenylbutyric acid from 2-oxo-4-phenylbutyric acid.

26. A process according to either claim 24 or claim 25 for the manufacture of 2-(R)-hydroxy-4-phenylbutyric acid from 2-oxo-4-phenylbutyric acid, wherein the spherical biocatalysts are employed in a fixed-bed reactor through which there is continuously passed an aqueous solution of the substrate 2-oxo-4-phenylbutyric acid, formate and an electron mediator.

## Revendications (Revendications pour l'(les) Etat(s) contractant(s) suivant(s): AT, BE, CH, DE, FR, GB, IT, LI, LU, NL, SE)

1. Biocatalyseur sous forme de Perles et à haute résistance mécanique, comprenant une substance à activité enzymatique, un polyélectrolyte cationique et des anions plurivalents, caractérisé en ce que le biocatalyseur contient de l'acide silicique et peut être obtenu à partir d'une suspension de la substance à activité enzymatique, de l'acide silicique, d'une solution tampon et du polyélectrolyte, la concentration finale de l'acide silicique étant dans une plage comprise entre 2 et 15 % (p/p).

2. Biocatalyseur sous forme de perles selon la revendication 1, caractérisé en ce que la substance à activité enzymatique consiste en cellules entières, fragments de cellules ou organites cellulaires d'un micro-organisme, en enzymes ou en une association quelconque de ces types de la substance à activité enzymatique.

3. Biocatalyseur sous forme de perles selon la revendication 1 ou 2, caractérisé en ce que la substance à activité enzymatique consiste en cellules entières d'une bactérie ou d'une levure.

4. Biocatalyseur sous forme de perles selon la revendication 3, caractérisé en ce que la bactérie appartient au genre *Proteus*.

5. Biocatalyseur sous forme de perles selon la revendication 4, caractérisé en ce que la bactérie appartient à l'espèce *Proteus vulgaris* et/ou à l'espèce *Proteus mirabilis.*

6. Biocatalyseur sous forme de perles selon la revendication 1 ou 2, caractérisé en ce que la substance à activité enzymatique contient une oxydoréductase ou est une oxydoréductase.

7. Biocatalyseur sous forme de perles selon l'une des revendications 1 à 6, caractérisé en ce que le polyélectrolyte cationique est le chitosane.

8. Biocatalyseur sous forme de perles selon la revendication 7, caractérisé en ce que le chitosane a une masse moléculaire d'environ 50 000 à environ 3 000 000.

9. Biocatalyseur sous forme de perles selon l'une des revendications 1 à 8, caractérisé en ce que les anions plurivalents sont des anions plurivalents minéraux.

10. Biocatalyseur sous forme de perles selon la revendication 9, caractérisé en ce que les anions minéraux sont un orthophosphate, des métaphosphates, des pyrophosphates, des polyphosphates ou des cyanoferrates.

11. Biocatalyseur sous forme de perles selon la revendication 10, caractérisé en ce que les anions minéraux sont le tripolyphosphate.

12. Biocatalyseur sous forme de perles selon l'une des revendications 1 à 8, caractérisé en ce que les anions plurivalents sont des anions plurivalents organiques.

13. Biocatalyseur sous forme de perles selon la revendication 12, caractérisé en ce que les anions organiques sont des carboxylates, sulfonates ou composés hydroxylés polymères organiques.

14. Biocatalyseur sous forme de perles selon l'une des revendications 1 à 13, caractérisé en ce que l'acide silicique est un acide silicique précipité.

15. Biocatalyseur sous forme de perles selon la revendication 14, caractérisé en ce que l'acide silicique précipité a une taille moyenne de particules dans la plage allant de 5 à 50 »m et une densité apparente moyenne dans la plage de 5 à 100 g/100 ml.

16. Procédé pour la préparation de biocatalyseurs sous forme de perles selon l'une des revendications 1 à 15, caractérisé en ce que l'on mélange une substance à activité enzymatique avec de l'acide silicique précipité sous forme solide, une solution aqueuse tampon et la solution aqueuse d'un polyélectrolyte cationique, en évitant la formation de mousse, pour obtenir une suspension, on introduit goutte à goutte la suspension dans un bain aqueux de réticulation contenant des anions plurivalents, et les perles de biocatalyseur résultantes sont rétrécies et solidifiées dans le bain de réticulation, et éventuellement séparées du bain de réticulation.

17. Procédé selon la revendication 16, caractérisé en ce qu'on évite la formation de mousse en effectuant le mélange en présence d'acide silicique.

18. Procédé selon la revendication 16 ou 17, caractérisé en ce que, comme polyélectrolyte cationique, on utilise du chitosane en solution aqueuse ayant une viscosité dans la plage allant de 1 000 à 20 000 cP.

19. Procédé selon l'une des revendications 16 à 18, caractérisé en ce que le rapport des parties en poids de l'acide silicique au polyélectrolyte cationique se situe dans une plage allant de 1,5:1 à 50:1.

20. Procédé selon l'une des revendications 16 à 19, caractérisé en ce que, dans la suspension de substance à activité enzymatique, acide silicique, solution tampon et polyélectrolyte cationique, la concentration finale du polyélectrolyte cationique se situe dans une plage allant de 0,3 à 1,5 % (p/p).

21. Procédé selon l'une des revendications 16 à 20, caractérisé en ce que l'anion plurivalent dans le bain de réticulation est le tripolyphosphate.

22. Procédé selon la revendication 21, caractérisé en ce que, pour le bain de réticulation, on utilise de l'acide tripolyphosphorique ou un sel de celui-ci, à une concentration dans la plage allant de 0,5 à 10 % (p/v).

23. Procédé selon la revendication 21 ou 22, caractérisé en ce que le volume du bain de réticulation est au moins le triple du volume de la suspension de substance à activité enzymatique, acide silicique, solution tampon et polyélectrolyte cationique.

24. Biocatalyseurs sous forme de perles et à haute résistance mécanique, caractérisés en ce qu'ils sont préparés selon un procédé conforme à l'une des revendications 16 à 23.

25. Procédé pour la conversion de substances organiques, caractérisé en ce que, pour la conversion, on utilise un biocatalyseur sous forme de perles selon l'une des revendications 1 à 15.

26. Procédé pour la préparation d'acides α-hydroxycarboxyliques et d'acides α-cétocarboxyliques, caractérisé en ce que, pour la réaction, on utilise un biocatalyseur sous forme de perles selon l'une des revendications 4 à 6.

27. Procédé selon la revendication 26, pour la préparation de l'acide 2-hydroxy-4-phénylbutyrique à partir de l'acide 2-oxo-4-phénylbutyrique.

28. Procédé selon la revendication 26 ou 27, pour la préparation de l'acide 2-(R)-hydroxy-4-phénylbutyrique à partir de l'acide 2-oxo-4-phénylbutyrique, caractérisé en ce que l'on utilise les biocatalyseurs sous forme de perles dans un'réacteur à lit fixe, dans lequel on fait passer en continu une solution aqueuse du substrat acide 2-oxo-4-phénylbutyrique, de formiate et d'un médiateur électronique.

## Revendications (Revendications pour l'(les) Etat(s) contractant(s) suivant(s): ES, GR)

1. Procédé pour la préparation d'un biocatalyseur sous forme de perles et à haute résistance mécanique, comprenant une substance à activité enzymatique, un polyélectrolyte cationique, des anions plurivalents et de l'acide silicique, caractérisé en ce qu'on mélange la substance à activité enzymatique avec de l'acide silicique précipité sous forme solide, une solution aqueuse tampon et la solution aqueuse d'un polyélectrolyte cationique, en évitant la formation de mousse, pour obtenir une suspension dans laquelle la concentration finale d'acide silicique se situe dans la plage comprise entre 2 et 15 % (p/p), on introduit goutte à goutte la suspension dans un bain aqueux de réticulation contenant des ions plurivalents, et les perles de biocatalyseur résultantes se rétrécissent et se solidifient dans le bain de réticulation, et sont éventuellement séparées du bain de réticulation.

2. Procédé selon la revendication 1, caractérisé en ce qu'on évite la formation de mousse en effectuant le mélange en présence de l'acide silicique.

3. Procédé selon la revendication 1 ou 2, caractérisé en ce que, comme polyélectrolyte cationique, on utilise du chitosane en solution aqueuse ayant une viscosité dans la plage allant de 1 000 à 20 000 cP.

4. Procédé selon l'une des revendications 1 à 3, caractérisé en ce que le rapport des parties en poids de l'acide silicique au polyélectrolyte cationique se situe dans la plage allant de 1,5:1 à 50:1.

5. Procédé selon l'une des revendications 1 à 4, caractérisé en ce que, dans la suspension de substance à activité enzymatique, acide silicique, solution tampon et polyélectrolyte cationique, la concentration finale du polyélectrolyte cationique se situe dans la plage allant de 0,3 à 1,5 % (p/p).

6. Procédé selon l'une des revendications 1 à 5, caractérisé en ce que l'anion plurivalent dans le bain de réticulation est le tripolyphosphate.

7. Procédé selon la revendication 6, caractérisé en ce que, pour le bain de réticulation, on utilise de l'acide tripolyphosphorique ou un sel de celui-ci, à une concentration dans la plage allant de 0,5 à 10 % (p/v).

8. Procédé selon la revendication 6 ou 7, caractérisé en ce que le volume du bain de réticulation est au moins environ le triple volume de la suspension de substance à activité enzymatique, acide silicique, solution tampon et polyélectrolyte cationique.

9. Procédé selon la revendication 1, pour la préparation d'un biocatalyseur sous forme de perles, caractérisé en ce que la substance à activité enzymatique du biocatalyseur consiste en cellules entières, fragments de cellules ou organites cellulaires d'un micro-organisme, en enzymes ou en une association quelconque de ces types de la substance à activité enzymatique.

10. Procédé selon la revendication 1, pour la préparation d'un biocatalyseur sous forme de perles, caractérisé en ce que la substance à activité enzymatique du biocatalyseur consiste en cellules entières d'une bactérie ou d'une levure.

11. Procédé selon la revendication 10, pour la préparation d'un biocatalyseur sous forme de perles, caractérisé en ce que la bactérie appartient au genre *Proteus.*

12. Procédé selon la revendication 11, pour la préparation d'un biocatalyseur sous forme de perles, caractérisé en ce que la bactérie appartient à l'espèce *Proteus vulgaris* et/ou à l'espèce *Proteus mirabilis.*

13. Procédé selon la revendication 1, pour la préparation d'un biocatalyseur sous forme de perles, caractérisé en ce que la substance à activité enzymatique du biocatalyseur contient une oxydoréductase ou est une oxydoréductase.

14. Procédé selon la revendication 1, pour la préparation d'un biocatalyseur sous forme de perles, caractérisé en ce que le biocatalyseur contient, en tant que polyélectrolyte cationique, du chitosane.

15. Procédé selon la revendication 14, pour la préparation d'un biocatalyseur sous forme de perles, caractérisé en ce que le chitosane a une masse moléculaire d'environ 50 000 à environ 3 000 000.

16. Procédé selon la revendication 1, pour la préparation d'un biocatalyseur sous forme de perles, caractérisé en ce que le biocatalyseur contient, en tant qu'anions plurivalents, des anions plurivalents minéraux.

17. Procédé selon la revendication 16, pour la préparation d'un biocatalyseur sous forme de perles, caractérisé en ce que les anions minéraux sont l'orthophosphate, des métaphosphates, pyrophosphates, polyphosphates ou cyanoferrates.

18. Procédé selon la revendication 17, pour la préparation d'un biocatalyseur sous forme de perles, caractérisé en ce que les anions minéraux sont le tripolyphosphate.

19. Procédé selon la revendication 1, pour la préparation d'un biocatalyseur sous forme de perles, caractérisé en ce que le biocatalyseur contient, en tant qu'anions plurivalents, des anions plurivalents organiques.

20. Procédé selon la revendication 19, pour la préparation d'un biocatalyseur sous forme de perles, caractérisé en ce que les anions organiques sont des carboxylates, sulfonates ou composés hydroxylés polymères organiques.

21. Procédé selon la revendication 20, pour la préparation d'un biocatalyseur sous forme de perles, caractérisé en ce que l'acide silicique du biocatalyseur est un acide silicique précipité.

22. Procédé selon la revendication 21, pour la préparation d'un biocatalyseur sous forme de perles, caractérisé en ce que l'acide silicique précipité a une taille moyenne de particules dans la plage allant de 5 à 50 »m et une densité apparente moyenne dans la plage de 5 à 100 g/100 ml.

23. Procédé pour la conversion de substances organiques, caractérisé en ce que, pour la conversion, on utilise un biocatalyseur sous forme de perles qui a été préparé selon un procédé conforme aux revendications 1 à 22.

24. Procédé pour la préparation d'acides α-hydroxycarboxyliques à partir d'acides α-cétocarboxyliques, caractérisé en ce que, pour la réaction, on utilise un biocatalyseur sous forme de perles qui a été préparé selon un procédé conforme aux revendications 11 à 13.

25. Procédé selon la revendication 24, pour la préparation de l'acide 2-hydroxy-4-phénylbutyrique à partir de l'acide 2-oxo-4-phénylbutyrique.

26. Procédé selon la revendication 24 ou 25, pour la préparation de l'acide 2-(R)-hydroxy-4-phénylbutyrique à partir de l'acide 2-oxo-4-phénylbutyrique, caractérisé en ce que l'on utilise les biocatalyseurs sous forme de perles dans un réacteur à lit fixe, dans lequel on fait passer en continu une solution aqueuse du substrat acide 2-oxo-4-phénylbutyrique, de formiate et d'un médiateur électronique.
